# EUROPÄISCHE PATENTSCHRIFT

(11) **EP 0 711 576 B1**
(45) Veröffentlichungstag und Bekanntmachung des Hinweises auf die Patenterteilung: **10.04.2002**
(21) Anmeldenummer: 95850198.3
(22) Anmeldetag: 08.11.1995
(51) Int. Cl.: A61N 1/05

(54) **Verfahren zur Herstellung von Elektroden für medizinische Anwendungen**
Method for producing electrodes for medical use
Procédé pour la production d'electrodes à usage médical

(30) Priorität: 11.11.1994 DE 4440386
(43) Veröffentlichungstag der Anmeldung: 15.05.1996
(73) Patentinhaber: St. Jude Medical AB, 175 84 Järfälla (SE)
(72) Erfinder: Lorenz, Hans Peter, D-90592 Schwarzenbrueck (DE); Strähler, Bernd, D-14109 Berlin (DE); Lindegren, Ulf, S-122 35 Enskede (SE); Ebert, Robby, D-09114 Chemnitz (DE); Illmann, Uwe, D-06118 Halle (DE); Reisse, Günter, D-09119 Chemnitz (DE)
(74) Vertreter: Kalling, Sven Owe

(56) Entgegenhaltungen:
- EP-A- 0 115 778
- EP-A- 0 191 238
- EP-A- 0 573 275
- EP-A- 0 596 589
- WO-A-93/00130

## Beschreibung

Die Erfindung betrifft ein Verfahren zur Herstellung von Elektroden für medizinische Anwendungen, insbesondere implantierbare Reizelektroden, mit einer aktiven Schicht aus porösem Titannitrid.

Elektroden der vorstehend genannten Art sind aus der europäischen Patentschrift 0 115 778 bekannt. Diese Elektroden bestehen aus einem elektrisch leitenden Trägermaterial und weisen im aktiven Bereich eine poröse Schicht aus einem Carbid, Nitrid oder Carbonitrid wenigstens eines der Metalle Titan, Vanadium, Zirkonium, Niob, Molybdän, Hafnium, Tantal oder Wolfram auf, d.h. unter anderem aus Titannitrid (TiN). Die bekannten Elektroden werden mittels eines PVD-Verfahrens (PVD = Physical Vapour Deposition) hergestellt, wobei die poröse Schicht durch physikalische Dampfabscheidung auf das als Substrat dienende Trägermaterial aufgebracht wird. Dazu wird das carbid-, nitrid- bzw. carbonitridbildende Metall, beispielsweise mittels eines Elektronenstrahlverdampfers, aus einem Vorrat des Metalls in einer stickstoff- und/oder methanhaltigen Atmosphäre verdampft und auf dem Substrat die entsprechende Metallverbindung abgeschieden.

Die auf die beschriebene Weise hergestellten Elektroden werden vorteilhaft als Reizelektroden für Herzschrittmacher eingesetzt. Für spezielle Anwendungszwecke ist nun beispielsweise aber eine Herzschrittmacher-Elektrode mit einer Elektrodenstruktur zweckmäßig, bei der zwei Bereiche - voneinander isoliert - konzentrisch zueinander angeordnet sind. Damit sich beim Kontakt mit der Körperflüssigkeit eine hohe Doppelschichtkapazität einstellt, ist es dann erforderlich, die Elektrode mit einer porösen Titannitridschicht zu versehen.

Eine derartige Elektrodenkonfiguration wurde mit Titannitrid-Elektroden bislang aber noch nicht realisiert.

Aufgabe der Erfindung ist es deshalb, ein Verfahren anzugeben, mit dem Titannitrid-Elektroden der eingangs genannten Art hergestellt werden können, bei denen die aktive Schicht strukturiert ist, d.h. eine bestimmte Elektrodenstruktur vorliegt.

Dies wird erfindungsgemäß durch ein Verfahren erreicht, bei dem ein Substrat aus elektrisch leitendem Material, das sich in einer titan-, stickstoff- und wasserstoffhaltigen Atmosphäre befindet, mittels eines Excimerlasers durch eine optische Maske bestrahlt wird, wobei auf dem Substrat eine Struktur aus porösem Titannitrid abgeschieden wird.

Mit dem Verfahren nach der Erfindung können beliebige Elektrodenkonfigurationen hergestellt werden, d.h. Elektroden mit beliebiger Struktur, beispielsweise eine Elektrodenanordnung mit zwei konzentrischen Elektroden. Hierbei wird nämlich eine entsprechende optische Maske mit Laserlicht beaufschlagt, wobei durch den Laserstrahl eine scharfe Abbildung der Maske in der Substratebene erfolgt, d.h. die Maske läßt Licht nur in der Form der gewünschten Struktur durch.

Würden Elektrodenstrukturen nach dem bislang angewendeten PVD-Verfahren hergestellt werden, dann müßte zunächst eine ganzflächige Beschichtung des Substrats erfolgen, und daran müßte sich ein auf das Titannitrid und das Substratmaterial abgestimmter Ätzvorgang - mit Photomaskierung - anschließen, um die gewünschte Elektrodenkonfiguration zu erzielen. Im Gegensatz dazu kann beim Verfahren nach der Erfindung, bei dem es sich um ein Laser-CVD-Verfahren (CVD = Chemical Vapour Deposition) handelt, d.h. um eine laserinduzierte chemische Gasabscheidung, eine bestimmte Elektrodenstruktur direkt in einem Arbeitsgang erzeugt werden, ohne daß nachfolgend ein Ätzprozeß erforderlich ist.

Beim Verfahren nach der Erfindung werden - auf photolytischem Weg - auf Substraten dünne Schichten aus Titannitrid lokal abgeschieden; das Substrat bleibt dabei unverändert. Die abgeschiedenen Schichten weisen eine Dicke bis zu 20 um und mehr auf, und sie besitzen eine rauhe Oberfläche, d.h. sie sind porös. Die Flächenkapazität dieser Schichten ist etwa um den Faktor 100 größer als diejenige von glatten Titanoberflächen. So beträgt die Flächenkapazität der Laser-CVD-TiN-Schichten nach der Erfindung beispielsweise bei einer Frequenz von 1 Hz etwa 2,5 mF/cm² (Titan: ca. 0,025 mF/cm²).

Zur Abscheidung des Titannitrids dient eine titan-, stickstoff- und wasserstoffhaltige Atmosphäre. Vorzugsweise weist diese Atmosphäre eine der folgenden Zusammensetzungen auf:
(a) Tetrakis(dimethylamino)-titan und Stickstoff sowie gegebenenfalls zusätzlich Wasserstoff;
(b) Titantetrachlorid, Stickstoff und Wasserstoff;
(c) Titantetrachlorid und Ammoniak.

Anstelle von Tetrakis(dimethylamino)-titan kann beispielsweise aber auch die entsprechende Ethylverbindung und anstelle von Titantetrachlorid beispielsweise auch Titantetrabromid eingesetzt werden. Ferner können Stickstoff/Wasserstoff-Gemische durch Ammoniak ersetzt werden und umgekehrt.

Das elektrisch leitende Substrat, auf dem die porösen TiN-Schichten abgeschieden werden, besteht vorzugsweise aus Titan. Daneben können beispielsweise aber auch Substrate aus Platin eingesetzt werden sowie aus anderen Edelmetallen, ferner Metallegierungen wie Elgiloy und nichtrostender Stahl, sogenannter VA-Stahl. Wichtig ist, daß das Substrat körperverträglich ist. Das Substrat kann sich im übrigen bei der Abscheidung auf erhöhter Temperatur befinden, beispielsweise auf einer Temperatur von ca. 60°C.

Beim Verfahren nach der Erfindung wird das Substrat vorzugsweise mit Licht einer der folgenden Wellenlängen bestrahlt: 193 nm, 248 nm, 308 nm oder 351 nm. Dies bedeutet, daß bevorzugt folgende Excimerlaser zum Einsatz gelangen:
ArF (193 nm), KrF (248 nm), XeCl (308 nm) und XeF (351 nm). Die Pulsfrequenz beträgt dabei im allgemeinen 100 Hz und die Pulslänge 20 ns.

Anhand eines Ausführungsbeispiels soll die Erfindung noch näher erläutert werden.

Als Substratmaterial dient Titan (Ti). Die Ti-Substrate werden unmittelbar vor der Beschichtung mit Ethanol und mittels eines Wasserstoff-HF-Plasmas gereinigt und in einer Reaktionskammer angeordnet, durch welche Tetrakis(dimethylamino)-titan, Stickstoff und Wasserstoff geleitet werden (Gesamtdruck: 10 mbar). Die Bestrahlung erfolgt mittels eines XeF-Excimerlasers (Wellenlänge: 351 nm; Pulsfrequenz: 100 Hz; Energieflußdichte: 162 mJ/cm²). Das Substrat kann auf eine Temperatur von 60°C geheizt werden.

Bei den genannten Abscheidebedingungen werden - bei einer Abscheidedauer von 20 min - TiN-Schichten mit einer Dicke von ca. 13 µm erhalten (Abscheiderate: 40 µm/h).

## Patentansprüche

1. Verfahren zur Herstellung von Elektroden für medizinische Anwendungen, insbesondere implantierbare Reizelektroden, mit einer aktiven Schicht aus porösem Titannitrid, **dadurch gekennzeichnet, daß** ein Substrat aus elektrisch leitendem Material, das sich in einer titan-, stickstoff- und wasserstoffhaltigen Atmosphäre befindet, mittels eines Excimerlasers durch eine optische Maske bestrahlt wird, wobei auf dem Substrat eine Struktur aus porösem Titannitrid abgeschieden wird.

2. Verfahren nach Anspruch 1, **dadurch gekennzeichnet, daß** sich das Substrat in einer Atmosphäre aus Tetrakis(dimethylamino)-titan und Stickstoff befindet.

3. Verfahren nach Anspruch 2, **dadurch gekennzeichnet, daß** zusätzlich Wasserstoff vorhanden ist.

4. Verfahren nach Anspruch 1, **dadurch gekennzeichnet, daß** sich das Substrat in einer Atmosphäre aus Titantetrachlorid, Stickstoff und Wasserstoff befindet.

5. Verfahren nach Anspruch 1, **dadurch gekennzeichnet, daß** sich das Substrat in einer Atmosphäre aus Titantetrachlorid und Ammoniak befindet.

6. Verfahren nach einem der Ansprüche 1 bis 5, **dadurch gekennzeichnet, daß** das Substrat aus Titan besteht.

7. Verfahren nach einem der Ansprüche 1 bis 6, **dadurch gekennzeichnet, daß** das Substrat mit Licht einer Wellenlänge von 193 nm, 248 nm, 308 nm oder 351 nm bestrahlt wird.

8. Verfahren nach einem der Ansprüche 1 bis 7, **dadurch gekennzeichnet, daß** sich das Substrat auf erhöhter Temperatur befindet.

## Claims

1. Method of manufacturing electrodes for medical applications, in particular implantable stimulating electrodes, with an active layer of porous titanium nitride, **characterised in that** a substrate of electrically conductive material, which is situated in a titanium-, nitrogen- and hydrogen-containing atmosphere, is irradiated through an optical mask by means of an excimer laser, whereby a structure of porous titanium nitride is deposited on the substrate.

2. Method as claimed in Claim 1, **characterised in that** the substrate is situated in an atmosphere of tetrakis (dimethyl amino)-titanium and nitrogen.

3. Method as claimed in Claim 2, **characterised in that** hydrogen is additionally present.

4. Method as claimed in Claim 1, **characterised in that** the substrate is situated in an atmosphere of titanium tetrachloride, nitrogen and hydrogen.

5. Method as claimed in Claim 1, **characterised in that** the substrate is situated in an atmosphere of titanium tetrachloride and ammonia.

6. Method as claimed in one of Claims 1 to 5, **characterised in that** the substrate comprises titanium.

7. Method as claimed in one of Claims 1 to 6, **characterised in that** the substrate is irradiated with light with a wavelength of 193 nm, 248 nm, 308 nm or 351 nm.

8. Method as claimed in one of Claims 1 to 7, **characterised in that** the substrate is at an increased temperature.

## Revendications

1. Procédé de production d'électrodes à usage médical, notamment d'électrodes implantables d'excitation ayant une couche active en nitrure de titane poreux, **caractérisé en ce qu'**il consiste à exposer à travers un masque optique un substrat en un matériau conducteur, qui se trouve dans une atmosphère contenant du nitrure de titane et de l'hydrogène, à un laser excimère en déposant sur le substrat une structure en nitrure de titane.

2. Procédé suivant la revendication 1, **caractérisé en ce que** le substrat se trouve dans une atmosphère de tétrakis(dimétahylamino)-titane et d'azote.

3. Procédé suivant la revendication 2, **caractérisé en ce qu'**il y a en outre de l'hydrogène.

4. Procédé suivant la revendication 1, **caractérisé en ce que** le substrat se trouve dans une atmosphère de tétrachlorure de titane, d'azote et d'hydrogène.

5. Procédé suivant la revendication 1, **caractérisé en ce que** le substrat se trouve dans une atmosphère de tétrachlorure de titane et d'ammoniac.

6. Procédé suivant l'une des revendications 1 à 5, **caractérisé en ce que** le substrat est en titane.

7. Procédé suivant l'une des revendications 1 à 6, **caractérisé en ce que** le substrat est exposé à de la lumière ayant une longueur d'onde de 193 nm, de 248 nm, de 308 nm ou de 351 nm.

8. Procédé suivant l'une des revendications 1 à 7, **caractérisé en ce que** le substrat se trouve à haute température.
